Europäisches Patentamt

(19) European Patent Office

Office européen des brevets

(11) Numéro de publication: **0 219 440 B1**

(12) **FASCICULE DE BREVET EUROPEEN**

(45) Date de publication de fascicule du brevet:
27.12.91

(51) Int. Cl.5: **A61L 2/02**, A61L 2/18, A61L 2/22

(21) Numéro de dépôt: 86420246.0

(22) Date de dépôt: 06.10.86

Le dossier contient des informations techniques présentées postérieurement au dépôt de la demande et ne figurant pas dans le présent fascicule.

(54) Procédé de décontamination d'une plage de sable ou de galets.

(30) Priorité: 08.10.85 FR 8515284

(43) Date de publication de la demande:
22.04.87 Bulletin 87/17

(45) Mention de la délivrance du brevet:
27.12.91 Bulletin 91/52

(84) Etats contractants désignés:
BE DE ES GB GR IT NL

(56) Documents cités:
EP-A- 0 115 893
US-A- 3 819 329

CHEMICAL ABSTRACTS, vol. 100, no. 23, 4 juin 1984, page 313, résumé no. 188657e, Columbus, Ohio, US; Yu.I. GRIGOR'EV et al.: "Effectiveness of using chlorine produced by electrolysis of sea water as a bactericide", & GIG. SANIT. 1984, (3), 84-6

"Le Dauphinè Libéré" lundi 6 Mars 1989: "Vers des plages propres"

"La Croix" Mardi 1e Juillet 1988, page 3

"VSD" 23/06/88 au 29/06/88 no. 564 , page 63.

(73) Titulaire: **F-TEC S.A.**
**Technoparc Gessien**
**F-01630 St-Genis-Pouilly(FR)**

(72) Inventeur: **Fresnel, Jean-Marie**
**2 hameau du Petit-Champs**
**Haut Thoiry F-01630 St-Genis-Pouilly(FR)**
Inventeur: **Bannochie, John Greig**
**70, Chemin Ami-Argand**
**CH-1290 Vemsoix(CH)**

(74) Mandataire: **Moinas, Michel**
**Cabinet Michel Moinas 13 Chemin du Levant**
**F-01210 Ferney-Voltaire(FR)**

## Description

La présente invention se rapporte à un procédé de décontamination d'une plage de sable ou de galets et se rapporte également à des installations pour la mise en oeuvre du procédé selon l'invention.

De nombreuses observations, notamment au niveau des médecins, ainsi que les analyses bactériologiques, laissent entrevoir une contamination du sable ou des galets des plages par des microorganismes pathogènes. Ces microorganismes peuvent être à l'origine de mycoses, d'affections urinaires ou cutanées. Leur présence peut être reliée à la promiscuité de personnes déjà contaminées et à la souillure du sable par les hommes ou les animaux domestiques, phénomènes fréquents en période de surfréquentation estivale. Interviennent également les matières fécales rejetées par des émissaires d'égout, parfois très voisins des plages.

Le sable dont la surface se contamine par l'importante fréquentation touristique retient l'humidité et la chaleur grâce à ses couches fines et denses et crée ainsi les conditions propices à la prolifération des germes microbiens.

On a ainsi mis en évidence une contamination importante des plages de sable, parfois pendant toute l'année, par des streptocoques, des staphylocoques, des coliformes et certains autres dermaphytes. Pour apprécier l'ampleur de ces phénomènes, on pourra se rapporter à un article intitulé "La Méditerranée dans un sale état" d'Edgard Gartner paru dans Sciences et Vie n° 815 du mois d'août 1985 (voir pages 16 et 20 notamment).

On peut noter çà et là quelques tentatives pour nettoyer ou désinfecter les plages, conduites précisément par des municipalités côtières de la mer Méditerranée. Certaines se contentent d'un lavage à l'eau, en espérant que par effet de lessivage, les germes pathogènes seront entraînés. D'autres ont essayé d'utiliser du sulfate d'ammonium quaternaire qui est un désinfectant bien connu, mais aussi un "engrais" très efficace qui, entraîné à la mer, va immanquablement entraîner une prolifération d'algues.

Le brevet US-A-3 819 239 décrit une installation de désinfection par pulvérisation de parois, d'appareils et de carcasses d'animaux, qui utilise une solution désinfectante à base d'acide hypochloreux comportant une cellule d'électrolyse de saumure.

D'un autre côté, il est décrit dans Chemical Abstracts, vol. 100, n° 23, 4 juin 1984, page 313, résumé n° 188657e, Columbus, Ohio, US ; Yu, I GRIGOR'EV et al. : "Effectiveness of using chlorine produced by electrolysis of sea water as a bactericide", & GiG. SANIT. 1984, (3), 84-6, l'utilisation d'une solution bactéricide formée par électrolyse d'eau de mer pour désinfecter diverses installations et appareillages, à une concentration de 250 à 450 mg de chlore/l.

Dans les deux exposés précités, il s'agit d'un traitement visant à désinfecter, c'est-à-dire à rendre aseptique, alors que, selon l'invention, on vise essentiellement à décontaminer, c'est-à-dire à éliminer les germes pathogènes tout en ménageant la faune indigène.

L'invention vise au contraire la désinfection, par des moyens biologiques, sans danger aucun pour les hommes et pour l'environnement. Ce traitement peut être d'ailleurs associé à un nettoyage classique, par des moyens mécaniques consistant par exemple à retourner le sable et à enlever toutes les souillures.

L'invention se rapporte à un procédé de décontamination d'une plage de sable ou de galets par traitement de la surface du sable ou des galets, dans lequel le traitement s'effectue par pulvérisation en surface d'un électrolysat d'eau de mer à des doses comprises entre 1 et 10 m³ de solution active par hectare de surface contaminée à traiter, l'électrolysat ayant une concentration d'agents actifs comprises entre 100 et 500 mg/l, exprimée en équivalent chlore.

Il faut observer ici que les valeurs de 250 à 450 mg de chlore par litre du document cité plus haut, et les valeurs de 100 à 500 mg/l selon l'invention sont en réalité sans aucune commune mesure. Dans le premier cas, ces valeurs se rapportent à une surface sensiblement lisse, tandis que selon l'invention, elles se rapportent à un matériau divisé, dont la surface réelle, celle qui est traitée, est de plusieurs ordres de grandeurs supérieurs à la surface apparente, non développée.

Les valeurs de concentration en agents actifs comprises entre 100 et 500 mg/l peuvent être obtenues directement par électrolyse d'eau de mer, ou alors ces valeurs résultent d'une dilution subséquente d'un e'lectrolysat de mer plus concentré.

On prélève de l'eau de mer qui va être restituée à la nature comme eau de mer, dans le plus grand respect de l'écologie. De plus un énorme avantage du procédé tient au fait ce que le cycle indiqué ci-avant peut être bouclé *in situ*.

Ainsi, de l'eau de mer du rivage est aspirée dans un réservoir où elle subit une électrolyse et elle est directement pulvérisée sur la plage à désinfecter.

De l'eau de mer pompée préalablement filtrée est envoyée à débit constant dans la cellule d'électrolyse équipée d'un ensemble d'électrodes métalliques recouvertes d'une couche électrocatalytique. Les électrodes sont alimentées en courant continu, courant délivré par exemple par une batterie rechargeable. Pour un débit d'eau fixé, la densité de courant est réglée pour que la concentration d'espèces actives corresponde à la demande.

L'électrolyseur comporte deux compartiments dans lesquels sont disposées deux ensembles d'électrodes, anodes et cathodes non séparées, les ensembles étant associés en série électrique. L'eau de mer entre dans le premier compartiment puis passe ensuite dans le second ; la moitié des ampères-heures est consommée dans chacun d'eux. Une bonne agitation dans chacun des compartiments permet un mélange homogène des espèces en solution.

La filtration préalable de l'eau de mer permet une économie considérable en espèces actives, puisqu'on élimine ainsi la matière organique présente dans l'eau de mer, qui réagirait avec celles-ci. On filtre par exemple sur charbon actif.

Les espèces actives produites par électrolyse sont essentiellement des ions hypochlorites et hypobromites, selon un schéma réactionnel qui, dans le cas de l'eau de mer, est le suivant :

$$2\ Cl^- \longrightarrow Cl_2\ +\ 2e^-\ \text{à l'anode}$$
$$2\ Na^+\ +\ 2\ H_2O\ +\ 2e^- \longrightarrow 2Na\ OH\ +\ H_2\ \text{à la cathode soit au total après mélange}$$
$$2\ Na\ Cl\ +\ H_2O \longrightarrow Na\ O\ Cl\ +\ Na\ Cl\ +\ H_2$$

La formation d'hypobromite peut s'effectuer selon le schéma

$$Cl\ O^-\ +\ Br^- \longrightarrow Br\ O^-\ +\ Cl^-$$

réaction rapide à des valeurs de pH inférieures à 8, ce qui est le cas de l'eau de mer.

Le pH de l'électrolysat reste sensiblement celui de l'eau de mer .

La quantité d'hypobromite formée reste faible par rapport au total puisqu'il n'y a qu'environ 68 ppm de bromure dans l'eau de mer. On a remarqué néanmoins que la présence d'hypobromite était très favorable à la qualité désinfectante de l'électrolysat, l'hypobromite semblant conduire à un effet synergique avec l'hypochlorite.

Les espèces actives, hypochlorites et hypobromites, seuls ou en mélange (voir ci-dessus), sont de puissants désinfectants et leur efficacité est limitée dans le temps par l'action des rayons U.V. (soleil). Ils redeviennent chlorures et bromures et le cycle naturel est ainsi bouclé.

De nombreux champignons et bactéries, staphylocoques et streptocoques sont les germes pathogènes les plus représentatifs d'une contamination bactérienne, en particulier :
- *Escherichia coli*
- *Streptococcus faecalis*
- *Staphylococcus aureus*
- *Candida* (nombreuses souches).

On a étudié sur une plage de sable 4 lots de 100 $m^2$ chacun pendant deux semaines sur lesquels ont été prélevés 3 échantillons par lot, selon le protocole suivant :
- un lot désinfecté une seule fois,
- un lot désinfecté toutes les semaines,
- un lot désinfecté tous les jours,
- un lot témoin qui ne subira aucune désinfection.

On a effectué au préalable 3 analyses sur chaque lot avant de commencer l'étude afin d'avoir une valeur moyenne de la contamination de la plage-testée.

De cette longue série d'essais, il est apparu qu'en période de surcharge estivale, la meilleure efficacité était obtenue par un traitement journalier, à raison de 1 à 10 $m^3$ d'eau électrolysée par hectare de plage, la concentration des espèces actives étant comprise entre 100 et 500 ppm, valeurs exprimées en équivalent de $Cl_2$. De préférence on traite autour de 2$m^3$/ha avec une concentration comprise entre 100 et 500 ppm. En période non estivale, on peut se contenter d'un traitement hebdomadaire.

On peut associer le traitement de pulvérisation de l'électrolysat avec des moyens mécaniques de retournement et tamisage du sable, de façon à désinfecter celui-ci en profondeur. En effet, en cas de grosses surcharges particulièrement, le sable est brassé par les usagers et les germes responsables des infections peuvent se retrouver à plusieurs centimètres ou dizaines de centimètres sous la surface.

Au plan des résultats, on a constaté une disparition totale des champignons et une diminution à 90 % au moins des staphylocoques et des streptocoques.

Il est possible d'aller, par ce traitement, jusqu'à une désinfection totale, c'est-à-dire jusqu'à l'aseptie.

D'une façon générale, on ne visera qu'une désinfection suffisante, dans les proportions indiquées plus haut. De la sorte, on détruit suffisamment de germes pathogènes pour que la plage devienne sans danger pour l'usager et on a toute chance de respecter les germes naturels, nécessaires à l'équilibre écologique et faisant barrière contre les germes pathogènes lorsque la plage n'est pas surexploitée

Parmi les nombreux avantages du procédé selon l'invention, appliqué au traitement des plages, on mettra plus particulièrement en évidence :
- l'utilisation d'eau de mer, donc pas de changement de milieu, et respect de l'écologie,
- la formation *in situ* d'espèces actives désinfectantes qui, après l'action de désinfection, disparaissent en se décomposant en produits d'origine (les sels contenus dans l'eau de mer) : il n'y a pas de pertubation de l'écosystème,
- pas d'apport de produits chimiques extérieurs, ni de concentrés à manipuler ; la sécurité est maximale et le coût est minimal ; le seul "produit" à consommer est l'électricité,
- l'odeur de la solution fraîche est agréable. Elle se confond à celle qu'on appelle "odeur de mer" et disparaît rapidement au soleil.
- combinaison possible avec un traitement mécanique de retournement du sable et d'élimination des souillures.

Bien entendu, l'électrolysat de mer peut être stocké, en bonbonnes par exemple, et être transporté pour des traitements à distance.

L'invention sera mieux comprise en référence au dessin annexé et à l'exemple qui suit, donnés à titre purement illustratif et non limitatif. Dans le dessin :
- la figure 1 est un schéma de principe illustrant la préparation de l'électrolysat d'eau de mer, et
- la figure 2 représente une installation mobile selon l'invention, destiné au traitement des plages, et
- la figure 3 représente une autre installation pour le traitement des plages, à poste fixe.

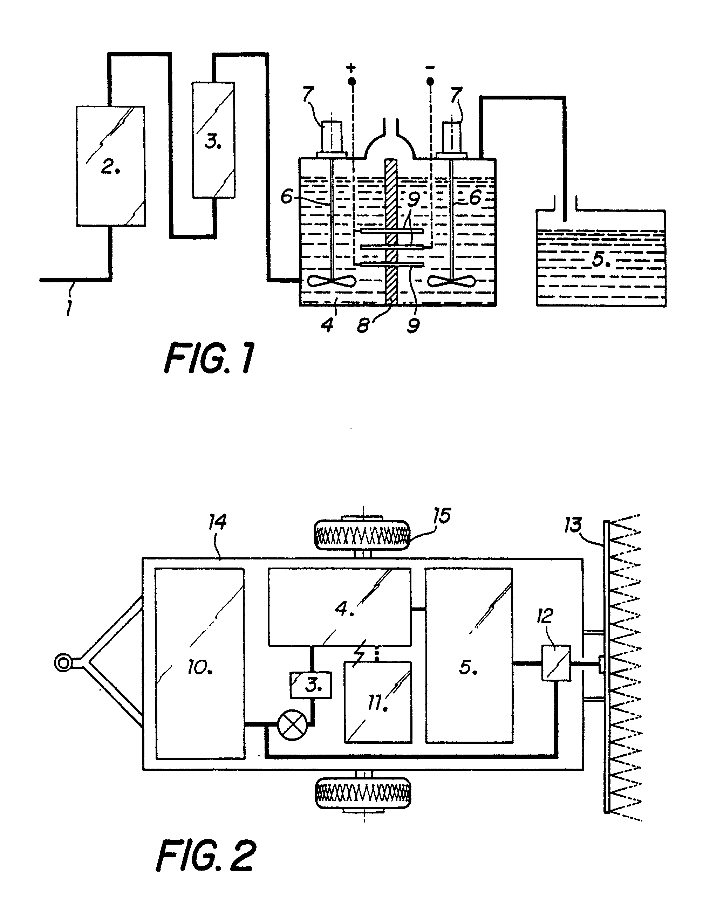

Comme on le voit sur la figure 1, l'eau de mer à électrolyser arrive par une entrée 1 et passe dans un filtre de charbon actif 2, pour retenir le plancton, les substances en suspension et les matières organiques. De là, l'eau de mer passe dans un débimètre 3 puis est envoyée dans l'électrolyseur. A la sortie de l'électrolyseur 4, l'eau de mer contenant les espèces actives est directement utilisée pour la pulvérisation ou envoyée dans un réservoir de stockage 5.

L'électrolyseur peut être avantageusement du type commercialisé sous la Marque Lectraqua® M1, M2 ou M5 par la Société F-TEC et qui est bien adapté pour la mise en oeuvre du procédé.

Comme on le voit sur la figure 1, cet électrolyseur 4 est muni d'agitateurs 6, d'un moteur 7 et comprend des parois étanches 8 séparant les compartiments. Les électrodes 9 sont du type DSA, qui sont des électrodes dimensionnellement stables en titane recouvertes d'un catalyseur.

L'électrolyseur est alimenté par une batterie de 12 V. Un réglage de la tension permet d'effectuer l'électrolyse sous 7,2 V avec une intensité de 7,5 A, ce qui correspond à une densité de courant de 4,4 A/dm$^2$ et à une consommation énergétique de 3,25 kwh/kg d'équivalent chlore produit. Si on ajoute la consommation électrique des agitateurs, la consommation totale est de 5 kwh/kg d'équivalent chlore produit.

Le dosage par l'iode de la quantité totale des espèces actives produites montre que pour un débit moyen de l'eau de mer de 33,3 l/h à la sortie de l'appareil, il est produit en moyenne 16,66 g d'équivalent chlore par heure. Ceci permet, par redilution avec de l'eau de mer de préparer les solutions de différentes concentrations, 200 ppm ou 400 ppm par exemple.

L'invention peut être mise en oeuvre à l'aide d'une installation mobile du type chariot, qui peut être traînée sur la plage par un tracteur, du genre de ceux qui véhiculent les engins mécaniques destinés à retourner le sable et à enlever les souillures. De la sorte, on peut combiner les deux traitements.

En variante, les traitements peuvent être combinés sur un seul et unique chariot associant le traitement mécanique et le traitement de désinfection.

Comme on le voit sur la figure 2, le chariot comprend un réservoir 10 rempli d'eau de mer et d'une capacité suffisante pour traiter de larges surfaces de sable. Si on le désire, on peut remplacer le réservoir 5 par un long conduit souple puisant l'eau directement dans la mer, correspondant à l'arrivée 1 de la figure 1.

On retrouve le débimètre 3 et l'électrolyseur 4 alimentés par des batteries 11. L'électrolysat est envoyé dans le réservoir de stockage 5 à partir duquel on peut préparer les solutions à pulvériser aux concentrations désirées.

Pour ce faire, le concentré provenant du réservoir 5 peut être redilué avec de l'eau de mer provenant du réservoir 10 ou de l'arrivée directe 1 et un bloc de commande 12 contrôle le taux de dilution.

A la sortie du bloc de commande 12, l'électrolysat est pulvérisé vers le sol à l'aide d'un pulvérisateur 13.

Comme on le voit sur la figure, le circuit emprunté par l'électrolysat est 10-3-4-5-12, tandis que l'eau de mer non traitée suit le circuit 10-12.

Tous les éléments précités sont ancrés solidement sur un plateau 14 muni de roues 15 afin de constituer un chariot qui peut être attelé à un tracteur.

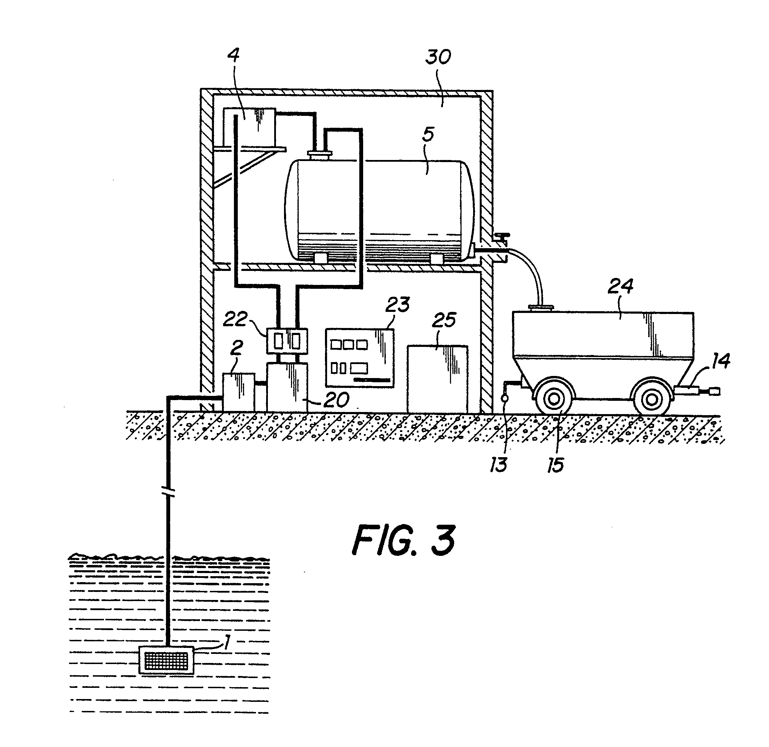

L'invention peut également être mise en oeuvre à l'aide d'une installation à poste fixe alimentant une ou plusieurs unités mobiles, comme on le voit sur la figure 3.

A cet effet, le local 30 est installé sur une plage ou à proximité d'une plage et est alimenté en eau de mer par un conduit 1 débouchant au large.

L'eau de mer est pompée jusqu'au local par une pompe 20 à travers un filtre 2 en charbon actif destiné à retenir le sable et les graviers. Ce filtre sert également à retenir les matières végétales en suspension, le plancton, ainsi que les matières organiques tels que les hydrocarbures. Un bloc de commande 22 permet le contrôle de l'ensemble, y compris la redilution de l'électrolysat.

Ce bloc de commande 22 est donc relié à l'électrolyseur 4 et à la cuve de stockage 5.

L'eau de mer envoyée dans l'électrolyseur 4 subit l'électrolyse, elle ressort à une concentration en "espèces actives" contrôlée à la fois par le débit et l'intensité du courant appliquée à l'électrolyseur.

Cet électrolyseur 4 est alimenté électriquement en 24 V continu par un transformateur-redresseur 25. Un tableau général 23 permet la surveillance et le contrôle de l'ensemble.

L'eau de mer électrolysée, ainsi que l'eau de mer brute, se retrouvent en mélange dans la cuve de stockage 5, d'où elles alimentent les unités mobiles.

Ces unités mobiles comprennent chacune une cuve d'épandage 24 montée sur un plateau 14 sur lequel est fixé un pulvérisateur 13. L'électrolysat stocké dans la cuve 24 est aspergé sur la surface à traiter au moyen de ce pulvérisateur 13. Des roues 15 permettent de véhiculer l'unité, par exemple au moyen d'un tracteur comme précédemment.

L'invention est également illustrée dans les exemples suivants, donnés à titre non limitatif.

Exemples 1 et 2

On traite deux plages de sable de la côte méditérranéenne en plein mois d'août, par la pulvérisation d'un électrolysat d'eau de mer, à raison de 2 m³/ha, l'électrolysat ayant une concentration en espèces actives de 200 ppm, respectivement 400 ppm.

La quantité d'électrolysat pulvérisée correspondrait à une épaisseur, en surface lisse, équivalente à 200 $\mu$m de liquide. En réalité, le sable étant un matériau finement divisé, le liquide se répartit sur une plus grande surface, donc sur une plus faible épaisseur.

Pour apprécier l'efficacité du traitement, on procède à des dosages en récoltant le sable sur une épaisseur de 1 à 2 cm, ce qui permet d'apprécier pleinement l'efficacité du traitement, si on se rapporte à l'épaisseur du film d'électrolysat indiqué ci-dessus.

Un échantillon de sable témoin prélevé juste avant le traitement sert d'élément de comparaison. Quant au sable traité, il est analysé 24 heures après l'application du traitement.

Les résultats obtenus sont les suivants :

```
1ère plage - exemple 1 - 200 ppm
```

| ! | : Escheri- | : Strepto- | : Staphylo- | : Candida ! |
|---|---|---|---|---|
| ! | : chia | : coccus | : coccus | : species ! |
| ! | : coli | : faecalis | : aureus | ! |
| !Sable : | | : | : | :9 C. pseudo- ! |
| !avant : | 34 | : 24 | : 10 | :tropicalis ! |
| !trai- : | | : | : | :7 C. albicans! |
| !tement: | | : | : | ! |
| !Sable : | | : | : | :3 C. pseudo- ! |
| !après : | 0 | : 0 | : 0 | :tropicalis ! |
| !trai- : | | : | : | :0 C. albicans! |
| !tement: | | : | : | ! |

2ème plage - exemple 2 - 400 ppm

| ! | : Escheri- | : Strepto- | : Staphylo- | : Candida ! |
|---|---|---|---|---|
| ! | : chia | : coccus | : coccus | : species ! |
| ! | : coli | : faecalis | : aureus | ! |
| !Sable : | | : | : | :13 C. pseudo-! |
| !avant : | 39 | : 34 | : 12 | :tropicalis ! |
| !trai- : | | : | : | :5 C. albicans! |
| !tement: | | : | : | ! |
| !Sable : | | : | : | :2 C. pseudo- ! |
| !après : | 0 | : 0 | : 0 | :tropicalis ! |
| !trai- : | | : | : | :0 C. albicans! |
| !tement: | | : | : | ! |

On constate donc la disparition des *Escherichia* et des staphylocoques pathogènes, ainsi que des *Candida albicans*. Quant aux *Candida pseudotropicalis*, seules de faibles proportions subsistes.

Exemple 3

On traite une autre plage de la côte méditérannéenne à l'aide d'une installation à poste fixe, telle que celle qui est représentée à la figure 3. La pulvérisation sur la plage est effectuée par les unités mobiles à la vitesse de 10 km/h sur 5 mètres de large, soit un traitement de 5 ha/h. La cuve d'épandage de l'unité mobile contient de 1 à 3 $m^3$ d'électrolysat. Le traitement est effectué à raison de 2 $m^3$ par ha de plage à décontaminer.

Pour traiter 5 ha de plage, il faudra une moyenne de 3,35 cuves, ce qui, compte-tenu des allers et retours et des temps de remplissage des éléments mobiles, correspond à environ 3 heures. De préférence, on effectue le traitement tôt le matin, avant l'arrivée des baigneurs, entre 5 et 8 heures.

L'électrolyseur fonctionne sous 24 V et 100 A avec un débit entrant de 6,3 l/mn. On obtiendra donc une concentration en équivalent Cl de 1,787 g/l. Par ailleurs, de l'eau de mer non électrolysée alimente la cuve de stockage à raison de 50 l/mn. On obtiendra ainsi après mélange, une concentration de 200 ppm d'espèces actives.

En trois heures, on produira ainsi 10 $m^3$ de solution, ce qui correspond à la quantité désirée pour traiter 5 ha de plage.

Le poste fixe comprend, dans son tableau général, un temporisateur mettant en route l'installation à 3 heures du matin pour l'arrêter à 6 heures. Un flotteur à contact dans la cuve, de 10$m^3$ par exemple, arrête le pompage d'eau électrolysée lorsque la cuve contient par exemple 9 $m^3$ de solution, ou bien s'il advient que les débits sont déréglés, ou encore si les prélèvements ne sont pas effectués entre 5 et 6 heures du matin.

Une autre sécurité placée dans l'électrolyseur arrête la circulation d'eau électrolysée si l'électrolyseur n'est pas entièrement rempli d'eau par suite d'une défaillance dans le pompage, une fuite, ou du développement d'une poche de gaz.

Exemple 4

Des essais de mouillage de sable sur une épaisseur de 25 cm ont été réalisés en laboratoire en répandant sur la surface 1,5 $cm^3$ d'électrolysat d'eau de mer coloré, 2 $cm^3$, 2,5 $cm^3$, 3 $cm^3$, 4 $cm^3$ par litre de sable, correspondant respectivement à 0,375 $l/m^2$ de surface de sable, 0,5 $l/m^2$, 0,625 $l/m^2$, 0,750 $l/m^2$, 1$l/m^2$. Ce sable traité par des colorants a ensuite été brassé énergiquement et on a pu constater la présence de sable mouillé par les colorants sur les 25 cm d'épaisseur.

Exemple 5

On a installé des rampes de pulvérisation sur une machine qui retourne le sable sur vingt à trente centimètres d'épaisseur, et qui crible et tamise le sable retourné pour retirer les détritus solides tels qu'excréments de chien, bâtons, papiers, cailloux, etc...

L'électrolysat est pulvérisé sur le sable en suspension au moment où celui-ci sort de la cribleuse . On constate alors que :
- d'une port que le sable est mouillé sur toute la profondeur retournée, entre 20 et 30 cm, soit en moyenne 25 cm,
- d'autre part que ce sable est désinfecté tant en surface qu'en volume sur la profondeur moyenne de 25 cm. Les germes présents, à raison de 7700/g de sable avant traitement (échantillon témoin), voient leur nombre ramené à 1400/g sable .

## Revendications

**1.** Procédé de décontamination d'une plage de sable ou de galets par traitement de la surface du sable ou des galets, caractérisé en ce que ce traitement s'effectue par pulvérisation en surface d'un électrolysat d'eau de mer à des doses comprises entre 1 et 10 $m^3$ de solution active par ha de surface contaminée à traiter, l'électrolysat ayant une concentration d'agent actif comprise entre 100 et 500 mg/l, exprimée en équivalent chlore.

**2.** Procédé selon la revendication 1, caractérisé en ce que la plage est une plage de sable.

**3.** Procédé selon la revendication 2, caractérisé en ce que la pulvérisation est réalisée alors que le sable est en cours de brassage.

**4.** Installation pour la mise en oeuvre du procédé selon la revendication 2, caractérisée en ce qu'elle

7

consiste en un chariot mobile alimenté en eau de mer et comprenant les moyens nécessaires à la préparation de l'électrolysat et à sa pulvérisation sur la surface à désinfecter.

5. Installation pour la mise en oeuvre du procédé selon la revendication 2, caractérisée en ce qu'elle comprend un poste fixe alimenté en eau de mer et comprenant les moyens nécessaires à la préparation de l'électrolysat, et des unités mobiles alimentées en électrolysat et permettant la pulvérisation de celui-ci sur la surface à désinfecter.

6. Installation selon la revendication 4 ou 5, caractérisée en ce qu'elle comprend des moyens mécaniques pour prélever le sable de la plage, le cribler et le brasser, la pulvérisation de l'électrolysat intervenant sur le sable lorsque celui-ci est brassé.

## Claims

1. A method of decontaminating a sand or pebble beach by treating the surface of the sand or pebbles, characterised in that the treatment is carried out by spraying on the surface an electrolysed solution of sea water in amounts of 1 to 10 m³ of active solution per hectare of contaminated surface to be treated, the concentration of active agent in the electrolysed solution being between 100 and 500 mg/l, expressed in chlorine equivalents.

2. The method according to claim 1, characterised in that the beach is a sand beach.

3. The method according to claim 2, characterised in that spraying is carried out while the sand is being turned over.

4. Installation for carrying out the method according to claim 2, characterised in that it includes a mobile cart supplied with sea water and comprising means adapted for producing the electrolysed solution and for spraying it on the surface to be disinfected.

5. Installation for carrying out the method according to claim 2, characterised in that it includes a permanently located station supplied with sea water and comprising means adapted for producing the electrolysed solution, and mobile units supplied with electrolysed solution and adapted for spraying the solution on the surface to be disinfected.

6. An installation according to claim 4 or 5, characterised in that it comprises mechanical means to take sand from the beach, sieve it and turn it over, spraying of the electrolysed solution taking place while the sand is being turned over.

## Patentansprüche

1. Verfahren zur Dekontamination eines sandigen oder steinigen Strandes durch Behandlung der sandigen oder steinigen Fläche, dadurch gekennzeichnet, daß diese Behandlung durch Zerstäubung eines Meerwasserelektrolysats auf die Fläche erfolgt in Dosierungen im Bereich von 1 bis 10 m³ aktiver Lösung pro ha (10000 m²) der zu behandelnden kontaminierten Fläche, wobei das Elektrolysat eine Konzentration des aktiven Mittels im Bereich von 100 bis 500 mg/l, ausgedrückt als Chloräquivalent, aufweist.

2. Verfahren nach Anspruch 1, dadurch gekennzeichnet, daß der Strand ein Sandstrand ist.

3. Verfahren nach Anspruch 2, dadurch gekennzeichnet, daß die Zerstäubung ausgeführt wird, während der Sand umgerührt wird.

4. Einrichtung zur Ausübung des Verfahrens nach Anspruch 2, dadurch gekennzeichnet, daß sie aus einem meerwasserbeschickten mobilen Wagen besteht und die notwendigen Mittel zur Erzeugung des Elektrolysats und seiner Zerstäubung auf die zu desinfizierende Fläche beinhaltet.

5. Einrichtung zur Ausübung des Verfahrens nach Anspruch 2, dadurch gekennzeichnet, daß sie aus einer meerwasserbeschickten festen Station besteht, die die notwendigen Mittel zur Zubereitung des Elektrolysats und bewegliche, mit Elektrolysat beschickte Einheiten beinhaltet, die dessen Zerstäubung auf die zu desinfizierende Fläche erlauben.

6. Einrichtung nach Anspruch 4 oder 5, dadurch gekennzeichnet, daß sie mechanische Mittel zum Entnehmen des Sandes vom Strand, sowie zum Sieben und zum Durchrühren des Sandes aufweist, wobei sich die Zerstäubung des Elektrolysats auf den Sand auswirkt, wenn dieser umgerührt wird.

FIG.1

FIG.2

FIG. 3